# EUROPEAN PATENT SPECIFICATION

(11) **EP 0 778 268 B1**
(45) Date of publication and mention of the grant of the patent: **03.11.1999**
(21) Application number: 96119534.4
(22) Date of filing: 05.12.1996
(51) Int. Cl.: C07C 313/06, C07C 311/09, C07C 303/36, A01N 47/02

(54) **Trifluoromethanesulfinanilide compound and use thereof**
Trifluormethansulfinanilidverbindung und ihre Anwendung
Composé de trifluorométhanesulfinanilidide et son utilisation

(30) Priority: 07.12.1995 JP 31885095; 05.07.1996 JP 17626696; 10.09.1996 JP 23959296
(43) Date of publication of application: 11.06.1997
(73) Proprietor: SUMITOMO CHEMICAL COMPANY LIMITED, Osaka-shi, Osaka 541-0041 (JP)
(72) Inventor: Mori, Tatsuya, Toyonaka-shi, Osaka (JP); Takada, Yoji, Toyonaka-shi, Osaka (JP)
(74) Representative: VOSSIUS & PARTNER

(56) References cited:
- CHEMICAL ABSTRACTS, vol. 98, no. 9, 28 February 1983 Columbus, Ohio, US; abstract no. 67132, XP002025600 & JP 57 156 407 A (SUMITOMO CHEMICAL CO LTD)
- J. FLUORINE CHEM., vol. 8, no. 5, 1976, pages 451-455, XP000196839 K.V. WERNER:
- SYNTHESIS, no. 11, 1987, pages 1015-1017, XP000196809 P. BROUGHAM ET AL:

## Description

The present invention relates to a certain trifluoromethanesulfinanilide compound and use thereof.

JP-A-57-156407 discloses that trifluoromethanesulfonanilides represented by the following formula [II] are useful as active ingredients of insecticides and acaricides. An object of the present invention is to provide an advantageous process for producing the compounds and production intermediates thereof.

This object could be achieved on the basis of the finding that the trifluoromethanesulfinanilide represented by the following formula [I] is a useful intermediate for the production of the trifluoromethanesulfonanilide represented by the following formula [II], and the trifluoromethanesulfinanilide
[I] can be efficiently converted into the trifluoromethanesulfonanilide
[II] by oxidizing with a magnesium monoperoxydicarboxylate.

That is, the present invention provides a trifluoromethanesulfinanilide compound represented by the formula: 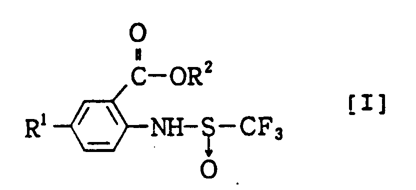 wherein R¹ represents a halogen atom (e.g. a chlorine atom or bromine atom), and R² represents a lower alkyl group (e.g. a C₁-C₄ alkyl group such as a methyl group or ethyl group) (hereinafter referred to as "the present compound"), and a process for producing a trifluoromethanesulfonanilide compound represented by the formula: 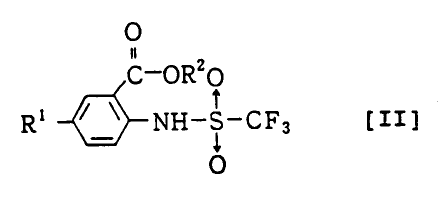 wherein R¹ and R² are as defined above, which comprises oxidizing the present compound with a magnesium peroxydicarboxylate (hereinafter referred to as "the present process).

It has also been found that the present compound has an excellent controlling activity against house dust mites.

That is, the present invention also provides a control agent for house dust mites, comprising the present compound as an active ingredient.

The present compound can be produced, for example, by reacting an aniline compound represented by the formula: 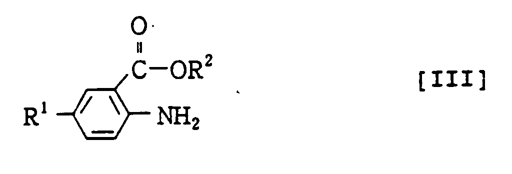 wherein R¹ and R² are as defined above with trifluoromethanesulfinyl chloride [CF₃S(O)Cl: it can be prepared by the process described in Chem. Ber. 107. 508 (1974)] or trifluoromethanesulfinic anhydride [(CF₃S(O))₂O: it can be prepared by the process described in Chem. Ber. 107. 508 (1974)] in the presence of a base.

This reaction is usually conducted in a solvent, and examples of the solvent used include aliphatic hydrocarbons such as n-hexane and n-heptane; alicyclic hydrocarbons such as cyclohexane and the like; aromatic hydrocarbons such as toluene, xylene and benzene; halogenated hydrocarbon such as methylene chloride, chloroform, carbon tetrachloride, 1,2-dichloroethane and chlorobenzene; ethers such as diisopropyl ether, tetrahydrofuran and dioxane; or a mixed solvent thereof.

Examples of the base used include organic bases such as pyridine, triethylamine, N,N-diethylaniline and N,N-dimethylaniline; alkali hydroxides such as sodium hydroxide and potassium hydroxide; and alkali carbonates such as sodium carbonate and potassium carbonate.

The reaction temperature is usually within the range from -20°C to the boiling point of the solvent used for the reaction or 100°C.

The molar ratio of the raw material and base which are used for the reaction, can be optionally set, but it is advantageous to react them in an equimolar ratio or a ratio approaching the equimolar ratio.

After completion of the reaction, the reaction solution is subjected to a conventional post-treatment such as extraction with organic solvent and concentration to isolate the desired present compound. The present compound obtained may be purified by a conventional operation such as recrystallization and chromatography according to the requirements.

The present compound can be also produced by reacting an aniline compound [III] with sodium trifluoromethanesulfinate [CF₃S(O)ONa: it can be prepared by the process described in JP-A- 3-56455] in the presence of an amine salt and thionyl chloride or phosgene.

This reaction is usually conducted in a solvent, and examples of the solvent used include aliphatic hydrocarbons such as n-hexane, and n-heptane; alicyclic hydrocarbons such as cyclohexane; aromatic hydrocarbons such as toluene, xylene and benzene; halogenated hydrocarbon such as methylene chloride, chloroform, carbon tetrachloride, 1,2-dichloroethane and chlorobenzene or a mixed solvent thereof.

Examples of the amine salt used include hydrochloride, p-toluene sulfonate and methane sulfonate of pyridine, triethylamine, N,N-diethylaniline and dimethylamine.

The reaction temperature is usually within the range from -20°C to the boiling point of the solvent used for the reaction or 100°C.

The molar ratio of the raw material and base which are used for the reaction, can be optionally set, but it is advantageous to react them in an equimolar ratio or a ratio approaching the equimolar ratio.

After completion of the reaction, the reaction solution is subjected to a conventional post-treatment such as extraction with organic solvent and concentration to isolate the desired present compound. The present compound obtained may be purified by a conventional operation such as recrystallization and chromatography according to the requirements.

The aniline compound represented by the formula [III] is commercially available, or it can be obtained according to the following production route: 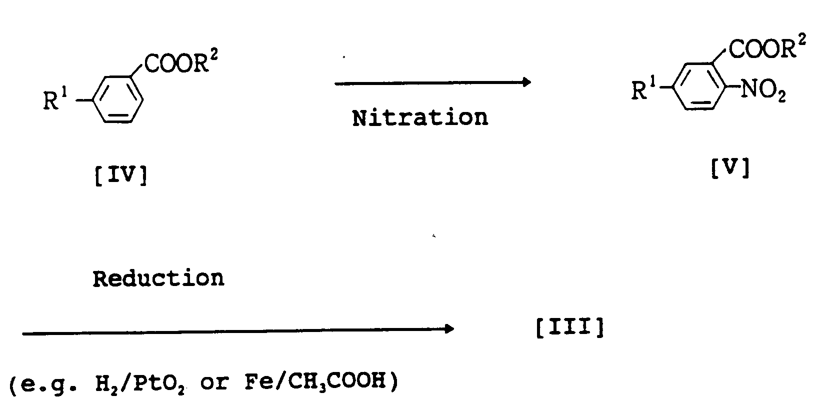 wherein R¹ and R² are as defined above.

The present compound can also be produced by oxidizing a trifluoromethanesulfenanilide compound represented by the formula: 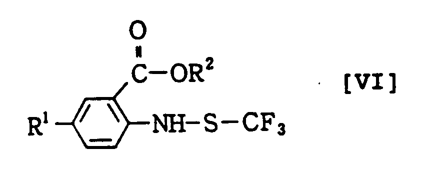 wherein R¹ and R² are as defined above with a peroxidated compound such as metachloroperbenzoic acid and magnesium monoperoxyphthalate. The oxidizing reaction is usually conducted in a solvent, and examples of the solvent used include halogenated hydrocarbon such as dichloromethane, chloroform, carbon tetrachloride, 1,2-dichloroethane and chlorobenzene when using metachloroperbenzoic acid as the peroxidated compound. When using magnesium monoperoxyphthalate as the peroxidated compound, examples of the solvent used include alcohols such as methanol and ethanol; water or a mixed solvent thereof. The reaction temperature is usually within the range from -20°C to the boiling point of the solvent used for the reaction or 100°C. The molar ratio of the raw materials and base which are used for the reaction, can be optionally set, but it is advantageous to react them in an equimolar ratio or a ratio approaching the equimolar ratio. After completion of the reaction, the reaction solution is subjected to a conventional post-treatment such as extraction with organic solvent and concentration to isolate the desired trifluoromethanesulfinanilide compound. The compound obtained may be purified by a conventional operation such as chromatography according to the requirements.

The trifluoromethanesulfenanilide compound represented by the formula [VI] can be produced, for example, by reacting the aniline compound represented by the above formula [III] with trifluoromethanesulfenyl chloride [CF₃SCl: it can be prepared by the process described in J. Org. Chem, 25. 2016 (1960)] in the presence of a base. This reaction is usually conducted in a solvent, and examples of the solvent used include aliphatic hydrocarbons such as n-hexane and n-heptane; alicyclic hydrocarbons such as cyclohexane; aromatic hydrocarbons such as toluene, xylene and benzene; halogenated hydrocarbon such as methylene chloride, chloroform, carbon tetrachloride, 1,2-dichloroethane and chlorobenzene; ethers such as diisopropyl ether, tetrahydrofuran and dioxane or a mixture thereof. Examples of the base used include organic bases such as pyridine and triethylamine; alkali metal hydroxides such as sodium hydroxide and potassium hydroxide; and alkali metal carbonates such as sodium carbonate and potassium carbonate. The reaction temperature is usually within the range from -20°C to the boiling point of the solvent used for the reaction or 100°C. The molar ratio of the raw materials and base, which are used for the reaction, can be optionally set, but it is advantageous to react them in an equimolar ratio or a ratio approaching the equimolar ratio. After completion of the reaction, the reaction solution is subjected to a conventional post-treatment such as extraction organic solvent, concentration and the like to isolate the desired trifluoromethanesulfenanilide compound [VI]. The compound obtained may be purified by a conventional operation such as chromatography according to the requirements.

The present process will be described hereinafter.

Examples of the magnesium monoperoxydicarboxylate used include a magnesium salt of a compound having a carboxyl group and a peroxycarboxyl group [-C(=O)OOH] at the ortho position of the benzene ring, such as magnesium monoperoxyphthalate; a magnesium salt of a compound having a carboxyl group and a peroxycarboxyl group at the vicinal position of the alicyclic hydrocarbon, such as magnesium monoperoxycyclohexane-1,2-dicarboxylate; and magnesium monoperoxymaleate. These magnesium monoperoxydicarboxylates are commercially available, or they can be produced by the process described in EP-A-27693.

The reaction is usually conducted in a solvent, and examples of the solvent used include alcohols such as methanol and ethanol, water or a mixed solvent thereof. The reaction temperature is usually within the range from -20°C to the boiling point of the solvent used for the reaction or 100°C The magnesium monoperoxydicarboxylate is usually used in an amount of 0.5 to 2 mol to one mol of the present compound.

The reaction is preferably conducted in the presence of a base, and examples of the base used include carbonates of alkali metals such as sodium carbonate and potassium carbonate; hydrogencarbonates of alkali metals such as sodium hydrogencarbonate and potassium hydrogencarbonate; and hydroxides of alkali metals such as sodium hydroxide, and potassium hydroxide. An amount of these bases used is usually from 0.9 to 1.2 mol, based on the amount of the magnesium monoperoxydicarboxylate.

After completion of the reaction, the reaction solution is subjected to a conventional post-treatment such as extraction with organic solvent, and concentration to isolate the desired trifluoromethanesulfonanilide compound of the formula [II]. The compound obtained may be purified by a conventional operation such as recrystallization and chromatography according to the requirements.

In the present invention, examples of the house dust mites include house dust mites (e.g. Dermatophagoides farinae Hughes) and grain mites (e.g. Tyrophagus putrescentiae).

For practical use of the present compound as an active ingredient of a control agent for house mites, the present compound may be used as it is without adding any other ingredients. The present compound is usually mixed with a solid carrier, a liquid carrier, a gaseous carrier or bait to formulate. Further, a surfactant and other additives may be added to the present compound according to the requirements. Examples of the formulations for the present compound include oil solutions, emulsifiable concentrates, wettable powders, flowables such as water suspensions and emulsions, granules, dusts, aerosols, heating fumigants such as combustible or chemical fumigant and porous ceramic fumigant, ULV agents, fog formulations (fogging agents), poison bait and mite-controlling sheet.

These formulations contain the present compound as an active ingredient in an amount of 0.01 to 95% by weight.

Examples of the solid carrier used for the formulation include fine powders or granules of clays (e.g. kaolin clay, diatomaceous earth, synthetic hydrated silicon dioxide, bentonite, Fubasami clay, acid clay), talcs, ceramics, other inorganic minerals (e.g. sericite, quartz, sulfur, active carbon, calcium carbonate, hydrated silica), and chemical fertilizers (e.g. ammonium sulfate, ammonium phosphate, ammonium nitrate, urea, ammonium chloride). Examples of the liquid carrier include water, alcohols (e.g. methanol, ethanol), ketones (e.g. acetone, methyl ethyl ketone), aromatic hydrocarbons (e.g. benzene, toluene, xylene, ethylbenzene, methylnaphthalene), aliphatic hydrocarbons (e.g. hexane, cyclohexane, kerosine, gas oil), esters (e.g. ethyl acetate, butyl acetate), nitriles (e.g. acetonitrile, isobutyronitrile), ethers (e.g. diisopropyl ether, dioxane), acid amides (e.g. N,N-dimethylformamide, N,N-dimethylacetamide), halogenated hydrocarbons (e.g. dichloromethane, trichloroethane, carbon tetrachloride), dimethyl sulfoxide and vegetable oils (e.g. soybean oil, cottonseed oil).

Examples of the gaseous carrier or propellant include CFCs (chlorofluorocarbons), butane gas, LPG (liquefied petroleum gas), dimethyl ether and carbon dioxide.

Examples of the surfactant includes alkyl sulfates, alkyl sulfonates, alkyl aryl sulfonates, alkyl aryl ethers, polyoxyethylenate thereof, polyethylene glycol ethers, polyhydric alcohol esters and sugar alcohol derivatives.

Examples of the adjuvant for formulation such as stocking agents or dispersing agents include casein, gelatin, polysaccharides (e.g. starch, gum arabic, cellulose derivatives, alginic acid), lignin derivatives, bentonite, sugars and synthetic water-soluble polymers (e.g. polyvinyl alcohol, polyvinyl pyrrolidone, polyacrylic acids). Examples of the stabilizer include PAP (acid isopropyl phosphate), BHT (2,6-di-tert-butyl-4-methylphenol), BHA (mixture of 2-tert-butyl-4-methoxyphenol and 3-tert-butyl-4-methoxyphenol), vegetable oils, mineral oils, surfactants, fatty acids and ester of fatty acids.

The base material of the combustible fumigant includes, for example, an exothermic agent such as a nitrate, nitrite, guanidine salt, potassium chlorate, nitrocellulose, ethylcellulose and wood powder; a pyrolytic stimulating agent such as an alkali metal salt, alkaline earth metal salt, dichromate and chromate; an oxygen source such as potassium nitrate; a combustion assistant such as melamine and wheat starch; a bulk filler such as diatomaceous earth; and a binding agent such as synthetic glue.

The base material of the chemical fumigant includes, for example, an exothermic agent such as a sulfide, polysulfide, hydrosulfide and water-containing salt of an alkaline metal; a catalytic agent such as a carbonaceous substance, iron carbide and active clay; an organic foaming agent such as azodicarbonamide, benzenesulfonylhydrazine, dinitrosopentamethylenetetramine, polystyrene and polyurethane; and a filler such as natural fibers and synthetic fibers.

The base material of the poisonous bait includes a bait material such as grain powder, vegetable oil, sugar and crystalline cellulose; an antioxidant such as dibutylhydroxytoluene and nordihydroguaiaretic acid; a preservative such as dehydroacetic acid ; a substance for preventing erroneous eating such as red pepper powder; and an attractant such as cheese flavor, onion flavor and peanut oil.

The formulations thus obtained are used as they are or diluted with water, and may be used simultaneously with other insecticides, other acaricides or synergists under non-mixed conditions or pre-mixed conditions.

Examples of the insecticides and/or acaricides include, for example, organophosphorous compounds such as Fenitrothion, Fention, Diazinon, Chlorpyriphos, Acephate, Methidachion, Disulfoton, DDVP, Sulprofos, Cyanophos, Dioxabenzofos, Dimethoate, Phenthoate, Malathion, Trichlorfon, Azinphosmethyl, Monocrotophos, and Ethion; carbamate compounds such as BPMC, Benfuracarb, Propoxur, Carbaril, Ethiofencarb, Aldicarb and Oxamyl; pyrethroid compounds such as Etofenprox, Fenvalerate, es-Fenvalerate, Fenpropathrin, Cypermethrin, Permethrin, Cyhalothrin, Deltamethrin, Cycloprothrin, d-Phenothrin, Tetramethrin, d-Allethrin, Resmethrin, Phthalthrin, Empenthrin, Prallethrin, Fluvalinate, Bifenthrin, Acrinathrin, Traromethrin and Silafluofen; thiadiazine derivatives such as Buprofezin; nitroimidazolidine derivatives such as Imidacloprid; Nereistoxin derivatives such as Cartap; benzoylphenylurea compounds such as Teflubenzuron and Fulphenoxron; thiourea derivatives such as Diafenthiuron; Debphenizide; 4-bromo-2-(4-chlorophenyl)-1-ethoxyethyl-5-trifluoromethylpyrrole-3-carbonitrile; phenyl salicylate; benzyl benzoate, paraoxybenzoate; formal iodide; phenols; phthalate; Methoxadiazon; and monoterpene epoxides.

When the present compound is applied as an active ingredient of the control agent for house dust mites, emulsifiable concentrates, wettable powders and flowables are usually diluted with water to the concentration of 0.01 to 10000 ppm. Oil solutions, aerosols, fumigants, fogging agents, ULV agents, poisonous baits and mite-controlling sheets are used as prepared.

The amount and concentration of application may be varied optionally according to the type of the formulations, timing, place, and method of application, the type of house dust mites, and the damage by house dust mites.

The following Examples further illustrate the present invention in detail but are not to be construed to limit the scope thereof.

Production Examples of the present invention are described hereinafter.

### Production Example 1

To a mixture of 2-methoxycarbonyl-4-chloro-trifluoromethanesulfenanilide [another name: 1,1,1-trifluoro-N-[4-chloro-2-(methoxycarbonyl)phenyl] methanesulfenamide] (produced in Reference Production Example 1 described hereinafter) (0.50 g, 1.8 mmol) and dried dichloromethane (10 ml) was added metachloroperbenzoic acid (manufactured by Wako Junyaku Co.,Ltd., purity: 70%) (0.47g, 1.9mmol) under ice cooling. After stirring at room temperature for 12 hours, the reaction solution was poured into iced water to obtain the dichloromethane layer. The aqueous layer was extracted once with dichloromethane and thus obtained dichloromethane layer was combined with the above dichloromethane layer. The combined layer was washed in turn with a saturated sodium thiosulfate solution and a saturated sodium hydrogencarbonate solution and dried over anhydrous magnesium sulfate, and then the solvent was distilled off under reduced pressure. The resultant residue was subjected to silica gel chromatography to obtain 0.17 g of 2-methoxycarbonyl-4-chloro-trifluoromethanesulfinanilide [another name: 1,1,1-trifluoro-N-[4-chloro-2-(methoxycarbonyl)phenyl] methanesulfinamide] (present compound (1)) (0.56 mmol, yield: 32%).
¹H-NMR (CDCl₃/TMS, δ (ppm)): 4.0 (3H, s), 7.3 (1H, d), 7.5 (1H, dd), 8.0 (1H, d), 10.5 (1H, brs)
m.p. 65.9°C.

### Production Example 2

To a mixture of methyl 5-chloro-2-aminobenzoate (0.66 g, 3.6 mmol), triethylamine (0.39 g, 3.9 mmol) and dried chloroform (10 ml) was added trifluoromethanesulfinyl chloride (0.60 g, 3.9 mmol) under ice cooling. [The trifluoromethanesulfinyl chloride was prepared as follows: To a mixture of potassium sulfite (19.3 g) and water (40 ml) was added dropwise trifluoromethanesulfonyl chloride (5.2 g) under ice cooling. After stirring at room temperature for 2 hours, the reaction solution was filtered. The residue obtained by concentrating the filtrate was extracted twice with heated acetone and the extracted solution was concentrated. The solid obtained by sufficiently drying the residue was heated at reflux, together with thionyl chloride (40 ml), for 2 hours. The reaction mixture was distilled under a normal pressure to obtain 1.3 g of trifluoromethanesulfinyl chloride.] After stirring at room temperature for one hour, the reaction solution was poured into ice water to obtain the chloroform layer. The aqueous layer was extracted once with chloroform and thus obtained chloroform layer was combined with the above chloroform layer. The combined layer was washed in turn with water and a saturated sodium chloride solution and dried over anhydrous magnesium sulfate, and then the solvent was distilled off under reduced pressure. The resultant residue was subjected to silica gel chromatography to obtain 0.64 g of 2-methoxycarbonyl-4-chloro-trifluoromethanesulfinanilide [another name: 1,1,1-trifluoro-N-[4-chloro-2-(methoxycarbonyl)phenyl]methanesulfinamide] (present compound (1)) (2.1 mmol, yield: 60%).

### Production Example 3

To a mixture of methyl 5-chloro-2-aminobenzoate (0.93 g, 5.0 mmol), triethylamine (0.56 g, 5.5 mmol) and dried chloroform (15 ml) was added trifluoromethanesulfinyl chloride (0.84 g, 5.5 mmol) under ice cooling. [The trifluoromethanesulfinyl chloride was prepared as follows: sodium trifluoromethanesulfinate (31.2 g, 200 mmmol) and thionyl chloride (47.6 g, 400 mmol) were mixed under ice cooling, followed by stirring at room temperature for 2 hours. The reaction mixture was distilled (b.p. 40-43°C) under a normal pressure to obtain 12 g of trifluoromethanesulfinyl chloride.] After stirring at room temperature for one hour, the reaction solution was poured into ice water to obtain the chloroform layer. The aqueous layer was extracted once with chloroform and thus obtained chloroform layer was combined with the above chloroform layer. The combined layer was washed in turn with water and a saturated sodium chloride solution and dried over anhydrous magnesium sulfate, and then the solvent was distilled off under reduced pressure. The resultant residue was subjected to silica gel chromatography to obtain 0.94 g of 2-methoxycarbonyl-4-chloro-trifluoromethanesulfinanilide [another name: 1,1,1-trifluoro-N-[4-chloro-2-(methoxycarbonyl)phenyl] methanesulfinamide] (present compound (1)) (3.1 mmol, yield: 62%).

### Production Example 4

To a mixture of methyl 5-chloro-2-aminobenzoate (0.93 g, 5.0 mmol), N,N-diethylaniline (0.82 g, 5.5 mmol) and dried toluene (15 ml) was added trifluoromethanesulfinyl chloride (0.84 g, 5.5 mmol) [the compound was prepared according to the process described in Production Example 3] under ice cooling. After stirring at room temperature for 3 hours, the reaction solution was poured into ice water to obtain the toluene layer. The aqueous layer was extracted once with toluene and thus obtained toluene layer was combined with the above toluene layer. The combined layer was washed in turn with water and a saturated sodium chloride solution and dried over anhydrous magnesium sulfate, and then the solvent was distilled off under reduced pressure. The resultant residue was subjected to silica gel chromatography to obtain 1.29 g of 2-methoxycarbonyl-4-chloro-trifluoromethanesulfinanilide [another name: 1,1,1-trifluoro-N-[4-chloro-2-(methoxycarbonyl)phenyl]-methanesulfinamide] (present compound (1)) (4.3 mmol, yield: 85%).

### Production Example 5

To a mixture of methyl 5-chloro-2-aminobenzoate (0.93g, 5.0mmol), pyridine hydrochloride (0.58g, 5.0mmol), sodium trifluoromethanesulfinate (0.78g, 5.0mmol) and dried toluene (15ml) was added thionyl chloride (0.60g, 5.0mmol) under ice cooling. After stirring at room temperature for 2 hours, the reaction solution was analyzed by gas chromatography [analysis conditions are as follows:
column: 10% DEXSIL 300GC (manufactured by Shimadzu Corp.)
column temperature: 170°C
injection temperature: 200°C
carrier gas and flow rate thereof: nitrogen gas, 50ml/minute
detection: FID] and, after confirming that the desired 2-methoxycarbonyl-4-chloro-trifluoromethanesulfinanilide was formed in a peak area percentage of gas chromatogram (hereinafter referred to as *"*the area percentage*"*) of 80%.

Other typical compounds of the present invention which can be produced according to the above Production Examples 1 to 5, and the numbers of the compounds are described hereinafter.
(2) 2-Methoxycarbonyl-4-bromo-trifluoromethanesulfinanilide
(3) 2-Ethoxycarbonyl-4-chloro-trifluoromethanesulfinanilide

Production Example of the trifluoromethanesulfenanilide compound represented by the formula [VI] is described hereinafter.

### Reference Production Example 1

To a mixture of methyl 5-chloro-2-aminobenzoate (0.98 g, 5.3 mmol), triethylamine (0.59 g, 5.8 mmol) and dried chloroform (10 ml) was added trifluoromethanesulfenyl chloride (0.79 g, 5.8 mmol) under ice cooling. [The trifluoromethanesulfenyl chloride was prepared as follows: To a mixture of trichloromethanesulfenyl chloride (9.3 g, 50 mmol) and dried sulfolane (40 ml) was added potassium fluoride (6.2 g, 100 mmol). While the reaction solution was heated to 50 to 200°C, trifluoromethanesulfenyl chloride formed was cooled and obtained (2.1g) by dry ice-acetone trap.] After stirring at room temperature for one hour, the reaction solution was poured into ice water to obtain the chloroform layer. The aqueous layer was extracted once with chloroform and thus obtained chloroform layer was combined with the above chloroform layer. The combined layer was washed in turn with water and a saturated sodium chloride solution and dried over anhydrous magnesium sulfate, and then the solvent was distilled off under reduced pressure. The resultant residue was subjected to silica gel chromatography to obtain 0.60 g of 2-methoxycarbonyl-4-chloro-trifluoromethanesulfenanilide [another name: 1,1,1-trifluoro-N-[4-chloro-2-(methoxycarbonyl)phenyl] methanesulfenamide] (2.1 mmol, yield: 40%).
¹H-NMR (CDCl₃/TMS, δ (ppm)): 3.9 (3H, s), 7.4 (1H, dd), 7.5 (1H, d), 7.9 (1H, d), 9.0 (1H, brs)
n_{D}^{25.0}1.5297

Examples of the present process are described hereinafter.

### Example 1

To a mixture of the present compound (1) (0.50 g, 1.7 mmol) and methanol (10 ml) was added magnesium monoperoxyphthalate haxahydrate (manufactured by Tokyo Kasei Kogyo Co., Ltd., purity: 95%) (0.86 g, 1.7 mmol) with stirring. After stirring at room temperature for 6 hours, the reaction solution was poured into ice water. The aqueous layer was extracted twice with chloroform and the chloroform layers were combined. The combined layer was dried over anhydrous magnesium sulfate, and then the solvent was distilled off under reduced pressure. The resultant residue was subjected to silica gel chromatography to obtain 0.26 g of 2-methoxycarbonyl-4-chloro-trifluoromethanesulfonanilide [another name: 1,1,1-trifluoro-N-[4-chloro-2-(methoxycarbonyl)phenyl] methanesulfonamide] (0.82 mmol, yield: 49%).
¹H-NMR (CDCl₃/TMS, δ (ppm)): 4.0 (3H, s), 7.5 (1H, dd), 7.7 (1H, d), 8.1 (1H, d), 11.0 (1H, brs)

### Example 2

To a mixture of the present compound (1) (0.50 g, 1.7 mmol) and methanol (10 ml) were added magnesium monoperoxyphthalate haxahydrate (manufactured by Tokyo Kasei Kogyo Co., Ltd., purity: 95%) (0.86 g, 1.7 mmol) and sodium carbonate (0.18 g, 1.7 mmol) with stirring. After stirring at room temperature for 6 hours, the reaction solution was analyzed by gas chromatography [analysis conditions are as follows (which are the same in the following Examples):
column: 10% DEXSIL 300GC (manufactured by Shimadzu Corp.)
column temperature: 170°C
injection temperature: 200°C
carrier gas and flow rate thereof: nitrogen gas, 50 ml/minute
detection: FID] and, after confirming that the desired 2-methoxycarbonyl-4-chloro-trifluoromethanesulfonanilide was formed in a peak area percentage of gas chromatogram (hereinafter referred to as "the area percentage") of 91%, the reaction solution was poured into ice water. The aqueous layer was extracted twice with chloroform and the chloroform layers were combined. The combined layer was dried over anhydrous magnesium sulfate, and then the solvent was distilled off under reduced pressure. The resultant residue was subjected to silica gel chromatography to obtain 0.44 g of 2-methoxycarbonyl-4-chloro-trifluoromethane-sulfonanilide (1.4 mmol, yield: 84%).

### Example 3

To a mixture of the present compound (1) (0.50 g, 1.7 mmol), methanol (7.5 ml) and water (2.5 ml) were added magnesium monoperoxyphthalate haxahydrate (manufactured by Tokyo Kasei Kogyo Co., Ltd., purity: 95%) (0.86 g, 1.7 mmol) and sodium carbonate (0.18 g, 1.7 mmol) with stirring. After stirring at room temperature for 6 hours, the reaction solution was analyzed by gas chromatography to confirm that the desired 2-methoxycarbonyl-4-chloro-trifluoromethanesulfonanilide was formed in the area percentage of 82%.

### Example 4

To a mixture of the present compound (1) (0.50 g, 1.7 mmol) and methanol (10 ml) were added magnesium monoperoxyphthalate haxahydrate (manufactured by Tokyo Kasei Kogyo Co., Ltd., purity: 95%) (0.86 g, 1.7 mmol) and sodium hydrogen carbonate (0.14 g, 1.7 mmol) with stirring. After stirring at room temperature for 6 hours, the reaction solution was analyzed by gas chromatography to confirm that the desired 2-methoxycarbonyl-4-chloro-trifluoromethanesulfonanilide was formed in the area percentage of 74%.

### Example 5

To a mixture of the present compound (1) (0.50 g, 1.7 mmol) and methanol (10 ml) were added magnesium monoperoxyphthalate haxahydrate (manufactured by Tokyo Kasei Kogyo Co., Ltd., purity: 95%) (0.86 g, 1.7 mmol) and sodium hydroxide (0.066 g, 1.7 mmol) with stirring. After stirring at room temperature for 6 hours, the reaction solution was analyzed by gas chromatography to confirm that the desired 2-methoxycarbonyl-4-chloro-trifluoromethanesulfonanilide was formed in the area percentage of 64%

Other typical compounds of the trifluoromethanesulfonanilide compound of the formula [II] which can be produced according to the above Examples, are described hereinafter.
2-Methoxycarbonyl-4-bromo-trifluoromethanesulfonanilide
2-Ethoxycarbonyl-4-chloro-trifluoromethanesulfonanilide

The embodiment wherein metachloroperbenzoic acid is used in place of magnesium monoperoxyphthalate in the process of the present invention is described hereinafter.

### Comparative Example 1

To a mixture of the present compound (1) (0.50 g, 1.7 mmol) and dried dichloromethane (10 ml) was added metachlroperbenzoic acid (manufactured by Wako junyaku Co., Ltd., purity: 70%) (0.45 g, 1.8 mmol) with stirring under ice cooling. After stirring at room temperature for 20 hours, the reaction solution was poured into ice water to obtain the dichloromethane layer. The aqueous layer was extracted once with dichloromethane and thus obtained dichloromethane layer was combined with above dichloromethane layer. The combined layer was washed in turn with a saturated sodium thiosulfate solution and a saturated sodium hydrogen carbonate solution and then dried over anhydrous magnesium sulfate, and then the solvent was distilled off under reduced pressure. The resultant residue was subjected to silica gel chromatography to obtain 0.16 g of 2-methoxycarbonyl-4-chloro-trifluoromethanesulfonanilide [another name: 1,1,1-trifluoro-N-[4-chloro-2-(methoxycarbonyl)phenyl] methanesulfonamide] (0.50 mmol, yield: 30%).

Formulation Examples are described hereinafter. In the description below, the present compounds are shown by the numbers of the above compounds and parts represent parts by weight.

### Formulation Example 1: Emulsifiable concentrates

Ten parts of each of the present compounds (1) to (3) are dissolved in 35 parts of xylene and 35 parts of dimethylformamide. Each of the obtained mixtures is mixed with 14 parts of polyoxyethylene styrylphenyl ether and 6 parts of calcium dodecylbenzenesulfonate, and stirred sufficiently to give 10% emulsifiable concentrate for each of the compound.

### Formulation Example 2: Wettable powders

Twenty parts of each of the present compounds (1) to (3) are added to a mixture of 4 parts of sodium laurylsulfate, 2 parts of calcium lignin sulfonate, 20 parts of synthetic hydrated silicon dioxide fine powder and 54 parts of diatomaceous earth, and the mixture is pulverized and mixed with a juice mixer to give 20% wettable powder for each compound.

### Formulation Example 3: Granules

5% Granules for each of the present compounds (1) to (3) are obtained by sufficiently mixing 5 parts of sodium dodecylbenzenesulfonate, 30 parts of bentonite and 60 parts of clay with each of the present compounds (1) to (3), kneading with an appropriate amount of water, granulating the mixture with a granulator, followed by air drying.

### Formulation Example 4: Dusts

One part of each of the present compounds (1) to (3) dissolved in an appropriate amount of acetone is mixed with 5 parts of synthetic hydrated silicon dioxide fine powder, 0.3 part of PAP and 93.7 parts of clay, and the mixture is pulverized and mixed with a juice mixer to give 1% dusts for each compound.

### Formulation Example 5: Flowables

Ten parts of each of the present compounds (1) to (3) are mixed with 40 parts of an aqueous solution containing 6 parts of polyvinyl alcohol, and the mixture is wet-pulverized to give a suspension. 10% Flowables for each compound is obtained by mixing the suspension with 40 parts of an aqueous solution containing 0.05 part of xantan gum and 0.1 part of aluminum magnesium silicate and then 10 parts of propylene glycol with stirring mildly.

### Formulation Example 6: Oil solutions

0.1% Oil solutions are obtained for each of the present compounds by dissolving 0.1 part of each of the present compounds (1) to (3) in 5 parts of xylene and 5 parts of trichloroethane, and mixing the solution with 89.9 parts of deodorized kerosine.

### Formulation Example 7: Oil-based aerosol

0.1 Part of each of the compounds (1) to (3), 0.2 part of tetramethrin, 0.1 part of d-phenothrin and 10 parts of trichloroethane in 59.6 parts of deodrized kerosine are mixed and the mixture is filled in an aerosol vessel. Then the vessel is set up with a valve, through which 30 parts of a propellant (liquefied petroleum gas) are charged under pressure to give each oil-based aerosol.

### Formulation Example 8: Water-based aerosol

0.2 Part of each of the compounds (1) to (3), 0.2 part of d-allethrin, 0.2 part of d-phenothrin, 5 parts of xylene, 3.4 parts of deodorized kerosine, and 1 part of an emulsifier [Atmos 300 (registered trade mark by Atlas Chemical)], and the mixture and 50 parts of pure water are filled in an aerosol vessel. Then the vessel is equipped with a valve, through which 40 parts of a propellant (liquefied petroleum gas) are charged under pressure.

### Formulation Example 9: Bait

A solution prepared by dissolving 10 mg of each of the present compounds (1) to (3) separately in 0.5 ml of acetone is mixed homogeneously with 5 g of solid bait powder for animals (Breeding Solid Feed Powder CE-2: trade name by Clea Japan Corp.). The acetone is removed by air-drying to obtain 0.5% poison bait for each compound.

### Formulation Example 10: Volatile agent

Volatile agent is prepared for each of the present compounds (1) to (3) by applying a solution prepared by dissolving 500 µg of each of the present compounds (1) to (3) in an appropriate amount of acetone, onto filter paper (2 cm x 2 cm, 0.3 mm in thickness) and removing the acetone by air-drying.

### Formulation Example 11: Mite-controlling sheet

A solution of each of the present compounds (1) to (3) in acetone is added dropwise to filter paper so that an amount of the compound is 1 g/1m² after being air-dried to remove the acetone to give mite-controlling sheet of each compound.

### Formulation Example 12: Heating fumigant

Heating fumigant of each of the present compounds (1) to (3) is prepared by impregnating a porous ceramic plate (4.0 cm x 4.0 cm, 1.2 cm in thickness) with a solution prepared by dissolving 100 mg of each of the present compounds (1) to (3) in an appropriate amount of acetone.

The following test example is performed to demonstrate that the present compound is useful as an active ingredient of a control agent of house dust mites. In the description below, the present compound is shown by the number of the above compound.

### Test Example 1: Test against mites (Tyrophagus putrescentiae and Dermatophagoides farinae Huges)

A piece of filter paper (diameter: 4 cm) was impregnated uniformly with an acetone solution of the present compound so that the amount of the impregnated compound was 0.8 g/m² after being air-dried. Approximately twenty heads of mites (Tyrophagus putrescentiae or Dermatophagoides farinae Huges) were put on the surface of the filter paper. An adhesive substance was applied on the circumference of the filter paper for preventing escape. After one day, the mortality was examined.

As a result, the present compound (1) showed the mortality of 100% against Tyrophagus putrescentiae and Dermatophagoides farinae Huges, respectively. In the non-treated filter paper, the mortality against Tyrophagus putrescentiae and Dermatophagoides farinae Huges was 0%, respectively.

## Claims

1. A trifluoromethanesulfinanilide compound represented by the formula: 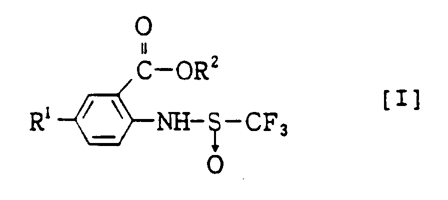 wherein R¹ represents a halogen atom, and R² represents a C₁-C₄ alkyl group.

2. A process for producing a trifluoromethanesulfonanilide compound represented by the formula: 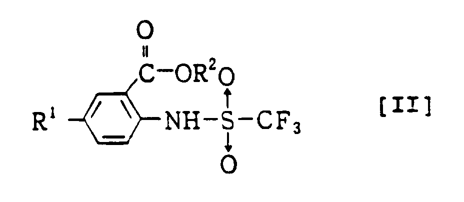 wherein R¹ represents a halogen atom, and R² represents a C₁-C₄ alkyl group, which comprises oxidizing the trifluoromethanesulfinanilide compound of claim 1 with a magnesium monoperoxydicarboxylate.

3. The process according to claim 2, wherein the reaction is conducted in the presence of a base.

4. The process according to claim 3, wherein the base comprises at least one selected from a carbonate, a hydrogencarbonate and a hydroxide of an alkali metal.

5. The process according to claim 3, wherein the base is a carbonate of an alkali metal.

6. The process according to claim 3, wherein the base is sodium carbonate.

7. The process according to claim 2, 3, 4, 5 or 6, wherein the magnesium monoperoxydicarboxylate is magnesium monoperoxyphthalate.

8. A controlling agent for house dust mites, comprising an effective amount of the trifluoromethanesulfinanilide compound according to claim 1 as an active ingredient.

9. A method for controlling house dust mites, which comprises applying an effective amount of the trifluoromethanesulfinanilide compound according to claim 1 to the house dust mites and/or a locus where the house dust mites inhabit.

10. Use of the trifluoromethanesulfinanilide compound according to claim 1 as an active ingredient of a controlling agent for house dust mites.

## Patentansprüche

1. Trifluormethansulfinanilidverbindung der Formel: 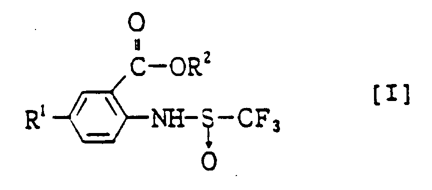 in der R¹ ein Halogenatom darstellt und R² einen C₁-C₄-Alkylrest darstellt.

2. Verfahren zur Herstellung einer Trifluormethansulfonanilidverbindung der Formel: 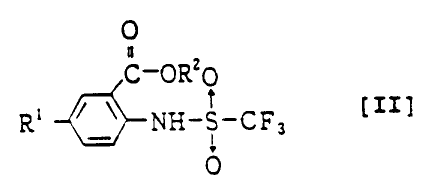 in der R¹ ein Halogenatom darstellt und R² einen C₁-C₄-Alkylrest darstellt, umfassend eine Oxidation der Trifluormethansulfinanilidverbindung nach Anspruch 1 mit einem Magnesiummonoperoxydicarboxylat.

3. Verfahren nach Anspruch 2, wobei die Reaktion in Gegenwart einer Base durchgeführt wird.

4. Verfahren nach Anspruch 3, wobei die Base mindestens eine Base umfaßt, ausgewählt aus einem Carbonat, einem Hydrogencarbonat und einem Hydroxid eines Alkalimetalls.

5. Verfahren nach Anspruch 3, wobei die Base ein Carbonat eines Alkalimetalls ist.

6. Verfahren nach Anspruch 3, wobei die Base Natriumcarbonat ist.

7. Verfahren nach Anspruch 2, 3, 4, 5 oder 6, wobei das Magnesiummonoperoxydicarboxylat Magnesiummonoperoxyphthalat ist.

8. Bekämpfungsmittel für Hausstaubmilben, umfassend eine wirksame Menge der Trifluormethansulfinanilidverbindung nach Anspruch 1 als Wirkstoff.

9. Verfahren zur Bekämpfung von Hausstaubmilben, umfassend das Aufbringen einer wirksamen Menge der Trifluormethansulfinanilidverbindung nach Anspruch 1 auf die Hausstaubmilben und/oder einen Ort, an dem die Hausstaubmilben leben.

10. Verwendung der Trifluormethansulfinanilidverbindung nach Anspruch 1 als Wirkstoff eines Bekämpffingsmittels für Hausstaubmilben.

## Revendications

1. Composé trifluorométhanesulfinanilide représenté par la formule : 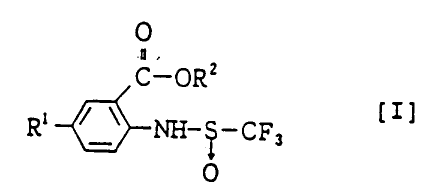 dans laquelle R¹ représente un atome d'halogène, et R² représente un groupe alkyle en C₁-C₄.

2. Procédé pour la production d'un composé trifluorométhanesulfonanilide représenté par la formule : 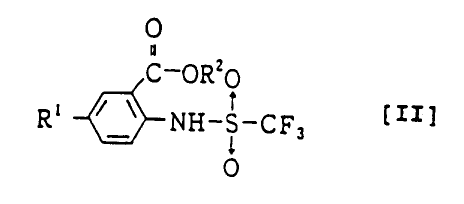 dans laquelle R¹ représente un atome d'halogène, et R² représente un groupe alkyle en C₁-C₄, qui comprend l'oxydation du composé trifluorométhanesulfinanilide de la revendication 1 avec un monoperoxydicarboxylate de magnésium.

3. Procédé selon la revendication 2, dans lequel la réaction est conduite en présence d'une base.

4. Procédé selon la revendication 3, dans lequel la base comprend au moins un composé choisi parmi un carbonate, un hydrogénocarbonate et un hydroxyde d'un métal alcalin.

5. Procédé selon la revendication 3, dans lequel la base est un carbonate d'un métal alcalin.

6. Procédé selon la revendication 3, dans lequel la base est le carbonate de sodium.

7. Procédé selon la revendication 2, 3, 4, 5 ou 6, dans lequel le monoperoxydicarboxylate de magnésium est le monoperoxyphtalate de magnésium.

8. Agent de lutte contre les acariens présents dans les poussières d'habitation, comprenant comme ingrédient actif une quantité efficace du composé trifluorométhanesulfinanilide selon la revendication 1.

9. Procédé de lutte contre les acariens présents dans les poussières d'habitation, qui comprend l'application d'une quantité efficace du composé trifluorométhanesulfinanilide selon la revendication 1 sur les acariens présents dans les poussières d'habitation et/ou à un endroit où habitent les acariens présents dans les poussières d'habitation.

10. Utilisation du composé trifluorométhanesulfinanilide selon la revendication 1 comme ingrédient actif ou agent de lutte contre les acariens présents dans les poussières d'habitation.
